# EUROPÄISCHE PATENTSCHRIFT

(11) **EP 0 452 740 B1**
(45) Veröffentlichungstag und Bekanntmachung des Hinweises auf die Patenterteilung: **18.10.1995**
(21) Anmeldenummer: 91105321.3
(22) Anmeldetag: 04.04.1991
(51) Int. Cl.: G01N 33/52, G01N 33/543

(54) **Testträger für die Analyse von Flüssigkeiten**
Test device to analyse fluids
Dispositif d'analyse de liquides

(30) Priorität: 14.04.1990 DE 4012216
(43) Veröffentlichungstag der Anmeldung: 23.10.1991
(73) Patentinhaber: BOEHRINGER MANNHEIM GMBH, 68298 Mannheim (DE)
(72) Erfinder: Krause, Manfred, W-6806 Viernheim (DE); Klein, Bernd, W-6700 Ludwigshafen (DE); Schindler, Gerhard, W-6718 Grünstadt 9 (DE); Schäfer, Peter, W-6700 Ludwigshafen (DE); Nötzel, Siegfried, Dr., W-6901 Wilhelmsfeld (DE)
(74) Vertreter: Pfeifer, Hans-Peter, Dr., Dr. H.-P. Pfeifer Dr. P. Jany, Patentanwälte

(56) Entgegenhaltungen:
- EP-A- 0 269 876
- EP-A- 0 334 015

## Beschreibung

Die Erfindung betrifft einen Testträger für die Analyse von Flüssigkeiten mit einem eine Testfeldöffnung umgebenden, aus mindestens zwei Teilen bestehenden Rahmen und einem in der Testfeldöffnung angeordneten Testfeld.

Zur qualitativen oder quantitativen analytischen Bestimmung von Bestandteilen von Flüssigkeiten, insbesondere Körperflüssigkeiten von Menschen oder Tieren, werden zunehmend sogenannte trägergebundene Tests verwendet. Bei diesen sind Reagenzien in den Testschichten eines insgesamt als Testträger bezeichneten Analyseelementes eingebettet. Sie werden mit der Probe in Kontakt gebracht. Die Reaktion von Probe und Reagenzien führt zu einem nachweisbaren Signal, insbesondere einer Farbänderung, welche visuell oder mit Hilfe eines Gerätes, meistens reflexionsphotometrisch, ausgewertet werden kann. Es sind zahlreiche verschiedene Testträgertypen bekannt, die sich nicht nur bezüglich der verwendeten Reagenzien, sondern auch durch ihren Aufbau, insbesondere hinsichtlich der Anordnung und Befestigung der Testschichten unterscheiden. Von besonderer praktischer Bedeutung sind folgende zwei Typen.

Streifenförmige Testträger des ersten Typs bestehen im wesentlichen aus einer länglichen Tragschicht aus Kunststoffmaterial und darauf angebrachten Testschichten. Die Verbindung zwischen den Testschichten und dem Kunststoffträger wird meist durch Kleben hergestellt, wobei die Verklebung vielfach nicht auf der ganzen Fläche, sondern nur an einer Kante der Testschicht erfolgt oder zusätzliche Befestigungsmittel (Netze oder Befestigungsfolien) an der Tragschicht befestigt sind, welche indirekt die Testschichten festhalten. Dadurch ist eine sehr vielfältige Anordnung der Testschichten nebeneinander und/oder übereinander je nach den Anforderungen des einzelnen Tests möglich. Die Testschichten können unabhängig voneinander hergestellt und bei der Endmontage des streifenförmigen Testträgers zusammengefügt werden.

Die EP-A-0 269 876 zeigt ein Beispiel der kombinierten Verwendung von Materialien unterschiedlicher Struktur, die keinen einstückigen Verbund bilden, als Testträger. Dabei liegt das aus einem Reaktionsmatrixkörper und einer gewebeartigen Basisschicht gebildete Testschichtpaket auf einem voluminösen Absorber vollflächig auf. Es ist keine Halterung des Testfeldpaketes in einem Rahmen vorgesehen. Der Absorberkörper ist als Andruckfläche für die Erzielung des Testschichtkontaktes erforderlich.

Die Erfindung richtet sich auf den eingangs erwähnten zweiten Testträgertyp, bei dem ein Testfeld ähnlich wie ein fotografisches Diapositiv von einem Rahmen gehalten wird. Sie werden im folgenden als "Testträger mit Rahmen" und in der englischsprachigen Literatur als "analysis slides" bezeichnet. Die Probe wird dabei üblicherweise durch die Testfeldöffnung auf das Testfeld aufgebracht. Nach Ablauf der Testreaktion kann die Farbbildung - meist auf der von der Probenseite abgewandten Seite des Testfeldes - beobachtet bzw. vermessen werden.

Bei diesem Testträgertyp werden bisher keine aus mehreren getrennten Testschichten bestehenden Testfelder verwendet. Vielmehr besteht das Testfeld üblicherweise aus einem durchsichtigen Kunststoffilm als Träger, welcher nacheinander mit verschiedenen schichtbildenden Flüssigkeiten beschichtet wird, in denen die Reagenzien oder Hilfsbestandteile für die Testdurchführung enthalten sind. Auch hier ist also ein mehrschichtiger Aufbau möglich, jedoch besteht das Testfeld aus einem einstückigen Schichtverbund, dessen Einzelschichten vollflächig miteinander verbunden sind. Dies ist erforderlich, um eine leichte Montage der Testfelder in dem Rahmen zu ermöglichen und vor allem um den gleichmäßigen Flüssigkeitskontakt zwischen den einzelnen Schichten sicherzustellen. Dieser gleichmäßige Flüssigkeitskontakt ist Voraussetzung für eine genaue Analyse, weil ein ungleichmäßiger Flüssigkeitstransfer zwischen den einzelnen Testschichten eine inhomogene Farbbildung zur Folge hat, die die Meßgenauigkeit erheblich beeinträchtigt. Dabei ist zusätzlich zu berücksichtigen, daß die einzelnen Schichten möglichst dünn sein sollen, um die erforderliche Menge an Probe und an Reagenzien möglichst klein zu halten und dennoch eine intensive Farbbildung zu erreichen.

Die Notwendigkeit, das Testfeld bei Testträgern mit Rahmen als einstückigen Schichtverbund herzustellen führt zu erheblichen Einschränkungen beim Einsatz dieses Testträgertyps. Die Entwicklung des Testschichtverbunds ist sehr aufwendig. Auch die Herstellung ist schwierig, weil die Genauigkeit der Analyse davon abhängt, daß jede einzelne Schicht mit sehr engen Toleranzen in gleichbleibender Qualität hergestellt wird. Dies ist naturgemäß schwerer zu realisieren, wenn die Schichten nacheinander in Form einer schichtbildenden Flüssigkeit aufgebracht werden, als wenn die Testschichten völlig getrennt voneinander hergestellt und erst bei der Endmontage des Testträgers zusammengefügt werden. Vor allem hat es sich als sehr vorteilhaft erwiesen, Testschichten mit sehr unterschiedlicher Struktur miteinander zu kombinieren. So haben beispielsweise Faserverbundstrukturen (Gewebe oder Vliese) feinporige Kunststoffschichten ("Membranen") und Partikelverbundstrukturen (z.B. EP-A-00 13 156) jeweils spezielle Eigenschaften, die für bestimmte Einsatzwecke vorteilhaft sind. Bei den bekannten Testträgern mit Rahmen ist die kombinierte Verwendung solcher unterschiedlicher Strukturen nur sehr eingeschränkt möglich.

Der Erfindung liegt daher die Aufgabe zugrunde, einen Testträger mit Rahmen zur Verfügung zu stellen, bei dem es möglich ist, das Testfeld aus mehreren getrennt voneinander hergestellten Testschichten zusammenzusetzen. Dabei soll selbstverständlich die Genauigkeit der Analyse nicht beeinträchtigt werden und eine rationelle Herstellung des Testträgers möglich sein.

Die Aufgabe wird bei einem Testträger der eingangs bezeichneten Art durch die Merkmale des kennzeichnenden Teils des Anspruchs 1 gelöst.

Im Rahmen der vorliegenden Erfindung wurde festgestellt, daß die Verwendung eines Testschichtpaketes mit mehreren, nicht vollflächig miteinander verbundenen Testschichten als Testfeld bei den bisher bekannten Testträgern mit Rahmen deshalb zu einem ungleichmäßigen Flüssigkeitstransfer zwischen den Testschichten und damit zu einer schlechten Analysegenauigkeit führt, weil die unvermeidlichen, wenn auch verhältnismäßig geringen, Dicketoleranzen üblicher Testfelder zu einer stark unterschiedlichen Druckbelastung des Testfeldpaketes führten. Es wurde gefunden, daß dieses Problem vermieden werden kann, wenn man dafür sorgt, daß der Druck, mit dem die Andruckfläche auf das Testschichtpaket drückt, im Toleranzbereich der Dicke des Testfeldpaketes im wesentlichen konstant ist. "Im wesentlichen konstant" ist dabei so zu verstehen, daß die Druckkonstanz und die davon abhängige Gleichmäßigkeit des Flüssigkeitstransfers zwischen den Testschichten die gewünschte Meßgenauigkeit gewährleisten muß. Der Druck der Andruckfläche auf das Testschichtpaket soll sich um weniger als 20% ändern, wenn sich dessen Dicke um 0,01 mm ändert.

Die gewünschte Elastizität läßt sich auf verschiedenerlei Weise erreichen. Beispielsweise können das Basisteil und/oder das Deckelteil selbst aus einem ausreichend elastischen Material gefertigt sein oder es können elastisch nachgiebige federnde Elemente (beispielsweise ein Andruckring aus einem hochelastischen Material) eingesetzt werden.

Besonders bevorzugte Ausführungsformen gehen jedoch davon aus, daß ein Kunststoff mit relativ geringer Eigenelastizität verwendet und die elastische Nachgiebigkeit durch entsprechende konstruktive Gestaltung von Deckelteil und/oder Basisteil einschließlich deren Verbindungselementen erreicht wird. Dadurch ist ein einfacher Aufbau mit kleinen Abmessungen möglich, das harte Rahmenmaterial ist wegen der Handhabungseigenschaften, insbesondere bei der vollmechanischen Verarbeitung der Testträger in entsprechenden Analyseautomaten, bevorzugt.

Solche bevorzugte konstruktive Maßnahmen und weitere Einzelheiten der Erfindung werden im folgenden anhand von in den Figuren schematisch dargestellten Ausführungsbeispielen erläutert; es zeigen:
- Fig. 1: Einen erfindungsgemäßen Testträger in perspektivischer Darstellung,
- Fig. 2: einen vergrößerten Querschnitt durch einen erfindungsgemäßen Testträger,
- Fig. 3: einen Längsschnitt durch einen erfindungsgemäßen Testträger,
- Fig. 4: einen vergrößerten Ausschnitt aus Fig. 3,
- Fig. 5: eine Aufsicht auf ein Basisteil und,
- Fig. 6: eine Aufsicht auf ein Deckelteil.
- Fig. 7: eine Aufsicht auf einen erfindungsgemäßen Testträger.

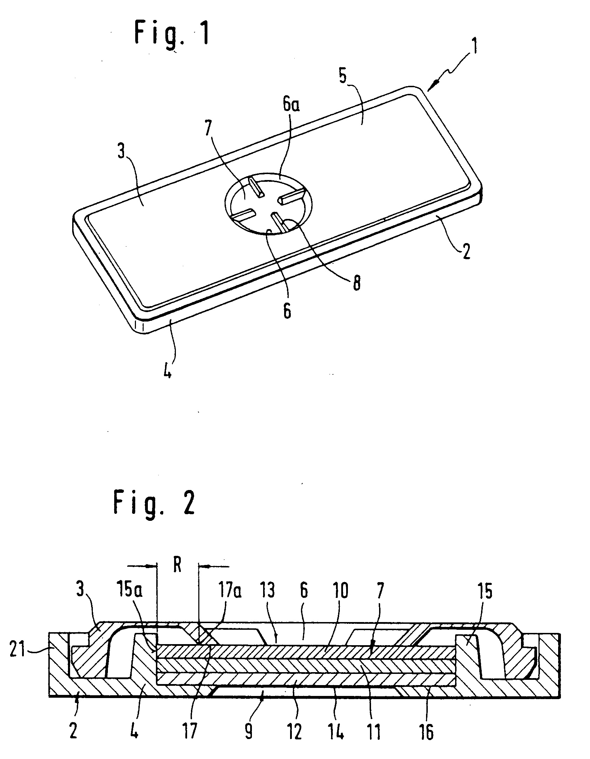

Der in Fig. 1 dargestellte Testträger 1 hat einen flachen, vorzugsweise weniger als 3mm, besonders bevorzugt nur etwa 2mm dicken Rahmen 2, welcher aus einem Deckelteil 3 und einem Basisteil 4 besteht. Im Zentrum der oberen Rahmenfläche 5 befindet sich eine Testfeldöffnung 6, durch welche das Testfeld sichtbar ist. Auf dem Testfeld liegen Andruckzungen 8 flach auf, welche vom Rand 6a der Testfeldöffnung 6 ausgehen.

Einzelheiten sind in den Figuren 2 bis 7 dargestellt.

Das Testfeld ist als Testschichtpaket 7 aus drei lose aufeinanderliegenden Testschichten 10,11,12 ausgebildet. Sie erfüllen verschiedene Funktionen im Testablauf. Beispielsweise kann die erste Testschicht 10 ein Abdecknetz sein, welches die darunterliegenden Schichten schützt und zugleich ein Netzmittel enthält, das die Ausbreitung der Probe fördert. Die Schicht 11 kann zur Abtrennung der Erythrozyten aus der Blutprobe dienen. Geeignet ist beispielsweise ein Glasfaservlies gemäß US-A-4 477 575. Die Schicht 12 kann beispielsweise ein Reagenzfilm sein, welche ein Reagenzsystem enthält, das zu einer für die Analyse charakteristischen farblichen Veränderung auf der Unterseite der Schicht 12 führt. Einzelheiten des Testablaufs sind für die vorliegende Erfindung nicht von Bedeutung. Im Regelfall ist der Testablauf aber so, daß die Probe auf einer Seite des Testschichtpaketes, welche als Probenaufgabeseite 13 bezeichnet werden kann, aufgegeben wird und die Messung bzw. visuelle Auswertung des Nachweissignals auf der anderen Seite des Testschichtpaketes (Nachweisseite 14) durch eine zweite Testfeldöffnung 9 erfolgt, welche üblicherweise die gleiche Größe wie die Testfeldöffnung 6 hat. Das Schichtpaket hat herstellungsbedingte Dickeschwankungen, die beispielsweise in der Größenordnung von plus/minus 0,02 mm liegen. Andererseits müssen die Schichten zuverlässig vollflächig aufeinanderliegen, damit ein gleichmäßiger Transfer der Probe von einer Schicht zur anderen erreicht wird. Selbst relativ geringe Unterschiede im Andruck führen, wie im Rahmen der vorliegenden Erfindung erkannt wurde, zu einer fleckigen Farbbildung. Es wurde festgestellt, daß bei Verwendung eines starren Rahmens mit Testträgern, deren Schichten an der Obergrenze des Toleranzbereiches liegen, ein unzureichender Flüssigkeitsdurchtritt festzustellen ist. Dies dürfte vermutlich auf eine störende Verdichtung einzelner Testschichten zurückzuführen sein. Bei einer Testpaketdicke an der unteren Grenze des Toleranzbereiches wird eine fleckige Farbbildung festgestellt, die auf einen ungleichmäßigen Flüssigkeitstransfer schließen läßt.

Das Schichtpaket 7 liegt in einer flachen Aufnahme 16, die in dem Basisteil 4 ausgeformt ist. Das Deckelteil 3 weist eine Andruckfläche 17 auf. Im dargestellten Fall umgibt die Andruckfläche die Testfeldöffnung ringförmig. Sie kann aber auch in anderer Weise ausgebildet sein. Wenn beispielsweise die Öffnung 6 so groß wie die Testschichten 12 oder sogar etwas größer sein soll, kann die Andruckfläche nur von der Unterseite von in die Testfeldöffnung hineinragenden Andruckzungen gebildet werden. Die Verwendung von Andruckzungen hat sich auch insoweit als vorteilhaft erwiesen, als durch Kapillarspalte zwischen der Unterseite der Andruckzungen und der obersten Testschicht 10 das Aufsaugen der Probe in die Testschicht gefördert wird.

Die Andruckfläche 17 liegt jedenfalls auf der obersten Testschicht 10 des Testschichtpakets 7 auf. "Oben" ist dabei als die dem Deckelteil zugewandte Seite zu verstehen. Dies muß nicht notwendigerweise die Probenaufgabeseite sein.

Die Aufnahme 16 ist im dargestellten Fall als von einer geschlossenen Wand umgebene Wanne ausgebildet. Zur Positionierung des Testschichtpaketes 7 können aber auch andere Mittel, insbesondere Stifte oder Wandstücke mit Zwischenräumen verwendet werden. Sie werden allgemein als Positioniereinrichtung 15 bezeichnet. Zwischen der dem Testschichtpaket 7 zugewandten inneren Begrenzung 15a der Positioniereinrichtung 15 und der äußeren Begrenzung 17a der Andruckfläche 17 ist vorzugsweise ein Ringspalt R vorgesehen, welcher groß genug ist, daß ein Ansaugen der Probenflüssigkeit durch Kapillarkräfte in den Ringspalt R vermieden wird. Vorzugsweise ist er mindestens 0,1 mm, besonders bevorzugt mindestens 0,3 mm, breit.

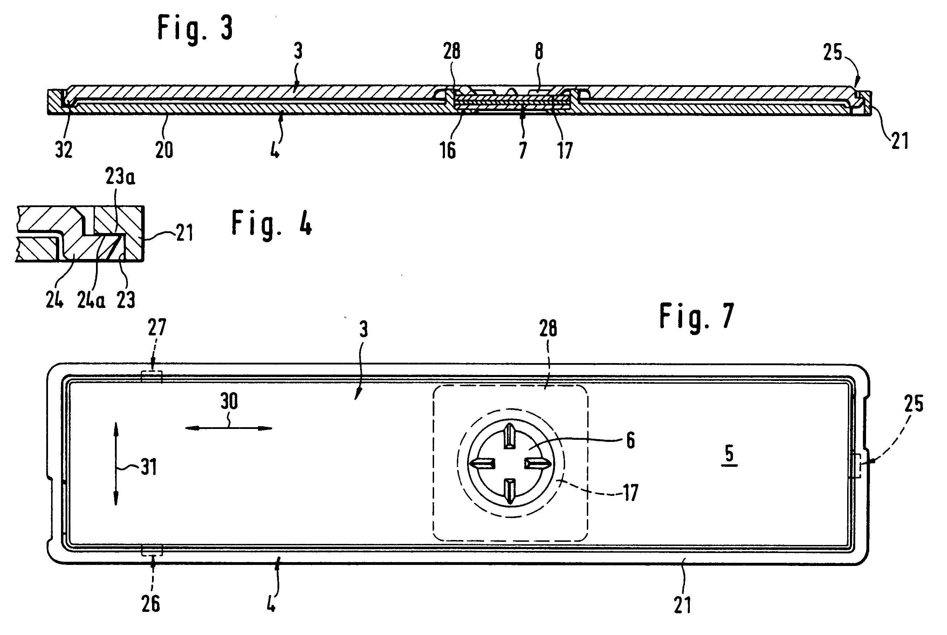

Zur Verbindung des Deckelteils 3 mit dem Basisteil 4 dienen vorzugsweise klebstofffreie diskrete Verbindungselemente 25,26,27, welche bevorzugt so ausgebildet sind, daß sie jeweils formschlüssig aneinander anliegende, die Relativbewegung senkrecht zur Ebene des Testschichtpaketes 7 begrenzende Begrenzungselemente 23a,24a (Fig. 4) aufweisen. Im dargestellten Fall hat das Deckelteil 3 einen Vorsprung 24, dessen obere Fläche eines der Begrenzungselemente bildet. Das Basisteil 4 hat Ausnehmungen 23, welche zum Zentrum des Rahmens 2 hin offen sind, und an denen die basisteilseitigen Begrenzungselemente 23a ausgebildet sind. Die Vorsprünge 24 greifen in die Ausnehmungen 23 ein. Je ein Vorsprung 24 und eine Ausnehmung 23 bilden ein Verbindungselement 25,26,27. Abweichende Gestaltungen der Verbindungselemente sind möglich, jedoch ist es vorteilhaft, wenn sie so ausgebildet sind, daß sie einerseits die Relativbewegung zwischen Deckelteil und Basisteil in Richtung senkrecht zu den Testschichten 10 bis 12 definiert begrenzen, während sie andererseits eine die elastische Deformierung von Deckelteil 3 und Basisteil 4 erleichternde geringfügige Relativbewegung beider Teile in Richtung parallel zur Fläche der Testschichten 10 bis 12 zulassen.

Bevorzugt sind die Verbindungselemente 25,26,27 als Clipsverbindung ausgebildet, wobei die formschlüssig ineinandergreifenden Teile 23,24 bei der Montage elastisch leicht verformt werden, um sie in Eingriff miteinander zu bringen. Die Clipsverbindung muß so gestaltet sein, daß sie sich bei der erfindungsgemäßen elastischen Verformung nicht lösen kann.

Bei dem dargestellten Ausführungsbeispiel hat das Basisteil 4 ein gegenüber seiner Bodenfläche 20 verdicktes Randprofil 21, an dem die Clipsverbindungen ausgebildet sind.

Im Hinblick auf die gewünschte Elastizität von Deckelteil und Basisteil ist es vorteilhaft, wenn beide nur mit verhältnismäßig wenigen diskreten klebstofffreien Verbindungselementen bezüglich einer Relativbewegung senkrecht zur Ebene des Testfelds fixiert sind und diese verhältnismäßig weit auseinanderliegen. Vorzugsweise sollte ihr Abstand mindestens dreimal so groß wie der Durchmesser der Testfeldöffnung 6 sein. Dadurch wird auch bei einem verhältnismäßig steifen Kunststoffmaterial eine ausreichende Elastizität erreicht. Die dargestellte rechteckige Rahmenform ermöglicht einen großen Abstand der Befestigungselemente bei geringer Gesamtfläche.

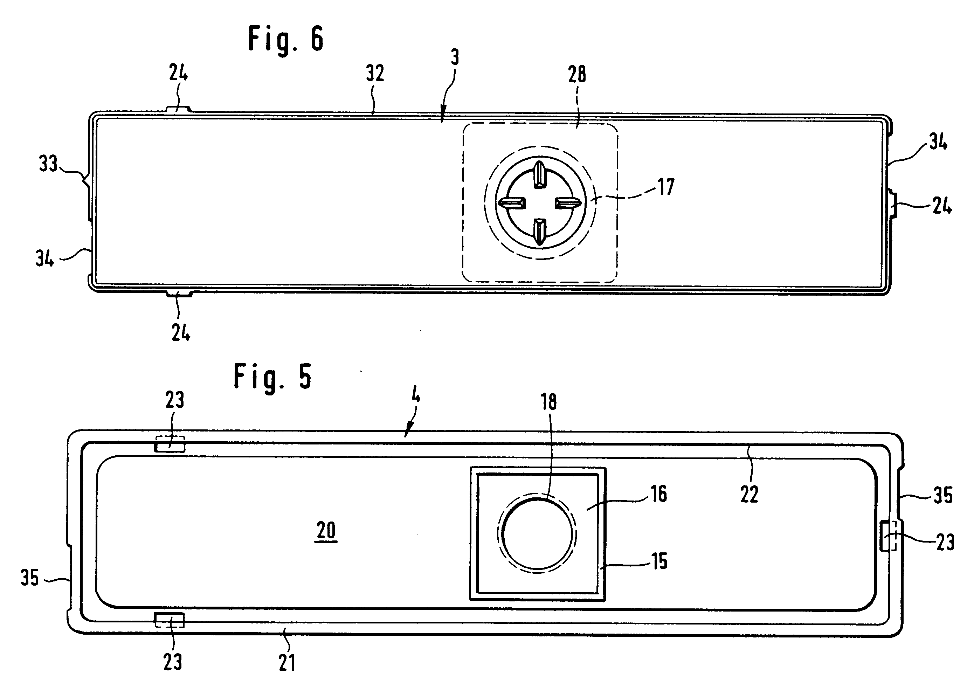

Alternativ oder zusätzlich kann die Elastizität, mit der die Andruckfläche 17 gegen das Testfeldpaket 7 gedrückt wird, dadurch gewährleistet werden, daß mindestens eines der Kunststoffprofilteile in der Umgebung der Testfeldöffnung 6 bzw. 9 eine besonders geringe Materialstärke hat. Im dargestellten bevorzugten Ausführungsbeispiel trifft dies für einen in den Figuren 6 und 7 gestrichelt dargestellten Elastizitätsbereich 28 zu. Die Materialstärke liegt hier vorzugsweise unter 0,3 mm, besonders bevorzugt unter 0,2 mm. Der Elastizitätsbereich 28 hat mindestens eine ebenso große Flächenausdehnung wie die Testfeldöffnung 6,9. Falls diese unterschiedlich sind, bezieht sich die Angabe auf die größere der beiden Testfeldöffnungen.

Für eine leichte Montage ist darüberhinaus vorteilhaft, daß die Positioniereinrichtung 15, welche die Aufnahme 16 in dem Basisteil 6 umgibt, höher als das Testschichtpaket 7 ist. Da sie vorzugsweise in dem Elastizitätsbereich 28 liegt, ist hierfür trotz geringer Gesamthöhe des Rahmens 2 genügend Raum vorhanden. Die hohe Positioniereinrichtung 15 stellt sicher, daß die Testschichten 10,11,12 trotz der bei der maschinellen Montage unvermeidlichen Erschütterungen nicht herausfallen.

In der Praxis ist auch wichtig, daß Deckelteil und Basisteil in Richtung ihrer in Fig. 7 durch die Pfeile 30 und 31 angedeuteten Flächendimensionen exakt zueinander positioniert sind. Zu diesem Zweck hat das Deckelteil eine schmale umlaufende Paßleiste 32, die an der Innenwand 22 des Randprofils 21 des Basisteils 20 anliegt. Die exakte Montage wird durch eine Positioniernase 33 erleichtert.

Das Deckelteil 3 und das Basisteil 4 sind vorzugsweise in Spritzguß aus Polystyrol oder ABS (Acrylnitril-Butadienstyrol) hergestellt. An ihren Rändern sind zweckmäßigerweise zurückspringende Ausnehmungen 34 bzw. 35 vorgesehen, an denen beim Herstellungsvorgang die Gießzapfen anstehen.

## Patentansprüche

1. Testträger für die Analyse von Flüssigkeiten mit einem eine Testfeldöffnung (6) umgebenden, aus mindestens zwei Teilen bestehenden Rahmen (2) und einem in der Testfeldöffnung (6) angeordneten Testfeld,
**dadurch gekennzeichnet,** daß
das Testfeld als Testschichtpaket (7) mit mindestens zwei aufeinander aufliegenden, nicht vollflächig miteinander verbundenen Testschichten (10,11,12) ausgebildet ist,
der Rahmen (2) zwei Profilteile aus Kunststoff aufweist, wobei
das erste Profilteil als Basisteil (4) dient und eine Aufnahme (16) für die Testschichten (10,11,12) aufweist,
das zweite Profilteil als Deckelteil (3) dient und eine auf der obersten Testschicht (10) des Testschichtpaketes (7) aufliegende Andruckfläche (17) aufweist und
der Rahmen (2) derartig elastisch gestaltet ist, daß sich der Druck der Andruckfläche (17) auf das Testschichtpaket (7) um weniger als 20% ändert, wenn sich die Dicke des Testschichtpaketes (7) um 0,01 mm ändert.

2. Testträger nach einem der vorhergehenden Ansprüche, **dadurch gekennzeichnet,** daß das Deckelteil (3) und Basisteil (4) mittels mindestens zwei Verbindungselementen (25,26,27) miteinander verbunden sind, welche jeweils formschlüssig aneinander anliegende, die Relativbewegung der Teile (3,4) senkrecht zur Ebene des Testschichtpaketes (7) begrenzende Begrenzungselemente (23a,24a) aufweisen.

3. Testträger nach Anspruch 2, **dadurch gekennzeichnet,** daß der Abstand der Verbindungselemente (25,26,27) mindestens drei mal so groß wie der Durchmesser der Testfeldöffnung (6,7) ist.

4. Testträger nach einem der Ansprüche 2 oder 3, **dadurch gekennzeichnet,** daß die Verbindungselemente (25,26,27) als Clipsverbindung ausgeführt sind.

5. Testträger nach Anspruch 4, **dadurch gekennzeichnet,** daß das Deckelteil (3) oder das Basisteil (4) ein gegenüber dem größten Teil seiner Fläche verdicktes Randprofil (21) hat und die Clipsverbindung in das Randprofil (21) eingreift.

6. Testträger nach einem der vorhergehenden Ansprüche, **dadurch gekennzeichnet,** daß mindestens eines der Kunststoff-Profilteile (4) einen Elastizitätsbereich (28) aufweist, der insgesamt mindestens eine ebensogroße Flächenausdehnung wie die Testfeldöffnung (6,7) und weniger als 0,3 mm, vorzugsweise weniger als 0,2 mm Materialstärke hat.

7. Testträger nach einem der vorhergehenden Ansprüche, **dadurch gekennzeichnet,** daß die Aufnahme (16) in dem Basisteil (4) von einer Positioniereinrichtung (15) für das Testschichtpaket (7) umgeben ist, welche höher als das Testschichtpaket (7) ist.

8. Testträger nach einem der vorhergehenden Ansprüche, **dadurch gekennzeichnet,** daß zwischen der äußeren Begrenzung (17a) der an dem Deckelteil (3) vorgesehenen Andruckfläche (17) und der dem Testschichtpaket (7) zugewandten inneren Begrenzung (15a) der Positioniereinrichtung (15) ein Ringspalt vorgesehen ist.

9. Testträger nach einem der vorhergehenden Ansprüche, **dadurch gekennzeichnet,** daß er Andruckzungen (8) aufweist, welche in die Testfeldöffnung (6) hineinragen.

## Claims

1. Test carrier for the analysis of fluids with a frame (2) consisting of at least two parts surrounding a test field opening (6) and with a test field disposed in the test field opening (6),
**characterised in that**
the test field is designed as a test layer package (7) with at least two superimposed test layers (10, 11, 12) not connected to one another over their whole area,
the frame (2) comprises two shaped pieces of plastics material, in which
the first shaped piece serves as base part (4) and comprises a seat (16) for the test layers (10, 11, 12),
the second shaped piece serves as lid part (3) and comprises a bearing surface (17) resting on the topmost test layer (10) of the test layer package (7) and
the frame (2) is designed with elastic properties so that the pressure with which the bearing surface (17) presses onto the test layer package (7) changes by less than 20% if the thickness of the test layer package (7) changes by 0.01 mm.

2. Test carrier according to any one of the preceding claims, **characterised in that** the lid part (3) and base part (4) are connected to one another by means of at least two connection elements (25, 26, 27) comprising limiting elements (23a, 24a) which butt positively against one another and limit the relative movement of the parts (3, 4) perpendicular to the plane of the test layer package (7).

3. Test carrier according to claim 2, **characterised in that** the spacing of the connection elements (25, 26, 27) is at least three times as great as the diameter of the test field opening (6, 7).

4. Test carrier according to any one of claims 2 or 3, **characterised in that** the connection elements (25, 26, 27) are designed as clip connections.

5. Test carrier according to claim 4, **characterised in that** the lid part (3) or the base part (4) has an edge profile (21) thickened in comparison with the greatest part of its surface and the clip connection engages with the edge profile (2).

6. Test carrier according to any one of the preceding claims, **characterised in that** at least one of the shaped plastics pieces (4) comprises an elasticity region (28) the surface are of which is at least as great as the test field opening (6, 7) and the material thickness is less than 0.3 mm, preferably less than 0.2 mm.

7. Test carrier according to any one of the preceding claims, **characterised in that** the seat (16) in the base part (4) is surrounded by a positioning means (15) for the test layer package (7) which is higher than the test layer package (7).

8. Test carrier according to any one of the preceding claims, **characterised in that** between the outer limitation (17a) of the bearing surface (17) provided on the lid part (3) and the inner limitation (15a), facing towards the test layer package (7), of the positioning means (15) an annular gap is provided.

9. Test carrier according to any one of the preceding claims, **characterised in that** it comprises bearing tongues (8) which project into the test field opening (6).

## Revendications

1. Support de test pour l'analyse de liquides, avec un cadre (2) constitué d'au moins deux parties, entourant un orifice de zone de test (6), et une zone de test disposée dans l'orifice de zone de test (6), caractérisé en ce que
la zone de test est constituée d'un empilement de couches de test (7), avec au moins deux couches de test (10, 11, 12) superposées, reliées entre elles seulement par une partie de leur surface,
le cadre (2) comprend deux profilés en matière plastique, le premier profilé servant de partie de base (4) et présentant une cavité (16) pour les couches de test (10, 11, 12), le second profilé servant de partie de couverture (3) et présentant une surface de pression (17) reposant sur la couche de test supérieure (10) de l'empilement de couches de test (7), et
le cadre est conçu sous forme élastique, de façon que la pression exercée par la surface de pression (17) sur l'empilement de couches de test (7) varie dans une proportion inférieure à 20 % lorsque l'épaisseur de l'empilement de couches de test (7) varie de 0,01 mm.

2. Support de test selon l'une quelconque des revendications précédentes, caractérisé en ce que la partie de couverture (3) et la partie de base (4) sont reliées entre elles par l'intermédiaire d'au moins deux éléments de liaison (25, 26, 27), qui présentent chacun des éléments de bordure (23a, 24a) superposés, en contact mécanique mutuel, délimitant le déplacement relatif des parties (3, 4) perpendiculairement au plan de l'empilement de couches de test (7).

3. Support de test selon la revendication 2, caractérisé en ce que la distance entre les éléments de liaison (25, 26 ,27) est au moins trois fois supérieure au diamètre de l'orifice de zone de test (6, 7).

4. Support de test selon l'une des revendications 2 ou 3, caractérisé en ce que les éléments de liaison (25, 26 ,27) sont réalisés sous forme de clip de serrage.

5. Support de test selon la revendication 4, caractérisé en ce que la partie de couverture (3) ou la partie de base (4) présente un profil de bordure (21) plus épais que la plus grande partie de sa surface, et le clip de serrage vient s'encliqueter dans le profil de bordure (21).

6. Support de test selon l'une quelconque des revendications précédentes, caractérisé en ce qu'au moins l'un des profilés (4) en matière plastique présente une zone d'élasticité (28) qui possède une superficie totale au moins aussi importante que l'orifice de zone de test (6, 7) et une épaisseur de matériau inférieure à 0,3 mm, de préférence inférieure à 0,2 mm.

7. Support de test selon l'une quelconque des revendications précédentes, caractérisé en ce que la cavité (16) de la partie de base (4) est entourée d'un dispositif de positionnement (15) pour l'empilement de couches de test (7), la hauteur de ce dispositif étant supérieure à celle de l'empilement de couches de test (7).

8. Support de test selon l'une quelconque des revendications précédentes, caractérisé en ce qu'entre la limite extérieure (17a) de la surface de pression (17) prévue sur la partie de couverture (3) et la limite intérieure (15a) du dispositif de positionnement (15), tournée vers l'empilement de couches de test (7), il est prévu une fente annulaire.

9. Support de test selon l'une quelconque des revendications précédentes, caractérisé en ce qu'il comporte des languettes de pression (8) qui pénètrent dans l'orifice de zone de test (6).
